# EUROPÄISCHE PATENTSCHRIFT

(11) **EP 1 536 703 B1**
(45) Veröffentlichungstag und Bekanntmachung des Hinweises auf die Patenterteilung: **13.09.2006**
(21) Anmeldenummer: 04739123.0
(22) Anmeldetag: 03.05.2004
(51) Int. Cl.: A24F 47/00

(54) **NICSTIC REFILL-SYSTEM**
NICSTIC REFILL SYSTEM
SYSTEME DE RECHARGE NICSTIC

(30) Priorität: 12.05.2003 DE 10321379
(43) Veröffentlichungstag der Anmeldung: 08.06.2005
(73) Patentinhaber: Nicstic AG, 6331 Hünenberg (CH)
(72) Erfinder: HOFFMANN, Thomas, 35460 Staufenberg (DE); PIROTH, Sylvia, 65199 Wiesbaden (DE)
(74) Vertreter: Fleck, Thomas
(86) Internationale Anmeldenummer: PCT/EP2004/004634
(87) Internationale Veröffentlichungsnummer: WO 2004/098324

(56) Entgegenhaltungen:
- EP-A1- 0 509 657
- DE-A1- 19 935 706
- DE-U1- 29 713 866
- US-A- 4 848 376
- US-A- 5 353 813
- US-A- 5 593 792
- US-B1- 6 532 965

## Beschreibung

Die Erfindung betrifft eine rauchfreie Zigarette (nachstehend NICSTIC REFILL-SYSTEM) nach dem Oberbegriff des Anspruches 1, die die beim üblichen Rauchen entstehenden Schadstoffe (Teer, kanzerogene Verbindungen wie Hydrazin, Chrysen, Arsen, Cadmium, Benzapyren, Benzaanthracen, Formaldehyd, Nitrosamine u.a.) vermeiden und dem Raucher nur das von ihm gewünschte Nikotin mit Aromastoffen zuführen soll. Eine derartige Zigarette ist aus der DE 199 35 706 bekannt. Auch ist bekannt bei ähnlichen Zigaretten eine aufgedampfte Beschichtung vorzusehen (siehe EP 0 509 657 A1).

Dabei war das erfindungsgegenständliche Ziel - neben der Nikotinzuführung - eine Annäherung soweit wie möglich an das normale Rauchen zu erreichen, nämlich durch
a) Beibehaltung der Form der Zigarette,
b) Beibehaltung des üblichen Filtermundstücks,
c) Schaffung des durch das Inhalieren entstehenden wohltuenden Wärmegefühls und
d) Beibehaltung des Tabakaromas
um dadurch den Wechsel von der normalen Zigarette auf das NICSTIC REFILL-SYSTEM für den Raucher zu erleichtern.

Durch die rauchfreie Nikotinzuführung kann der Benutzer des NICSTIC REFILL-SYSTEMs in immer größerem Umfange entstehenden Rauchverbotszonen (Flugzeuge, Bahnhöfe, öffentliche Gebäude, Restaurants), aber auch im häuslichen Bereich (Wohnzimmer, Kinderzimmer) seinen Nikotinbedarf decken, ohne Dritte (Passivraucher) zu belästigen und/oder zu schädigen und ohne gegen Verbote zu verstoßen.

Durch die Möglichkeit, variable Stärken des Nikotindepots anzubieten, kann der NICSTIC REFILL-SYSTEM in idealer Weise zur Rauch-Nikotin-Entwöhnung eingesetzt werden.

Dieses Ziel wird durch die im Anspruch 1 gekennzeichnete Zigarette gelöst.

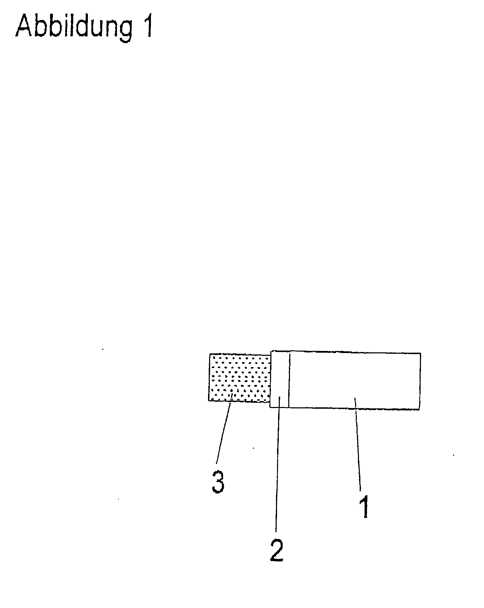

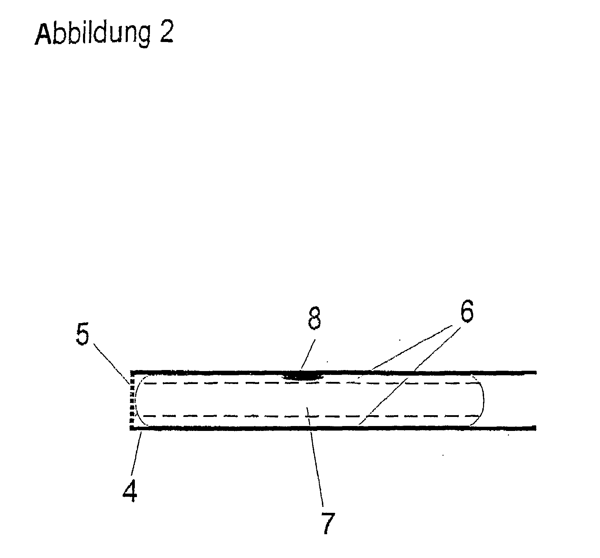

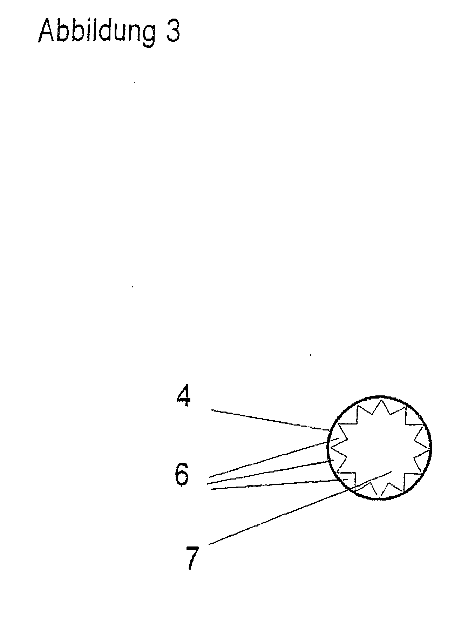

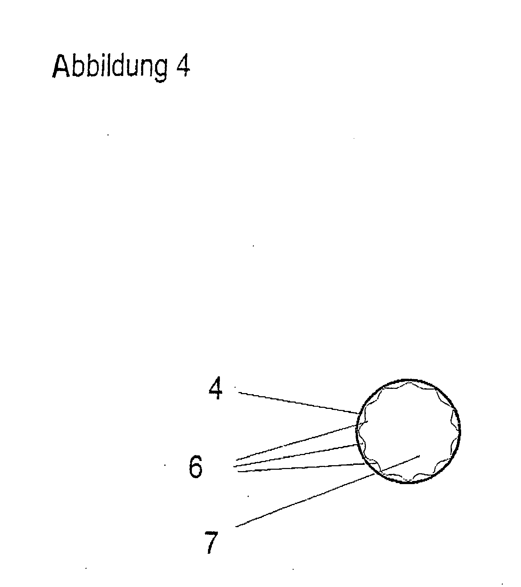

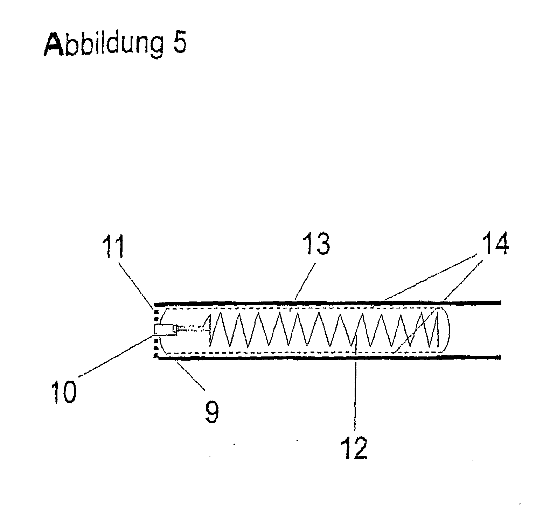

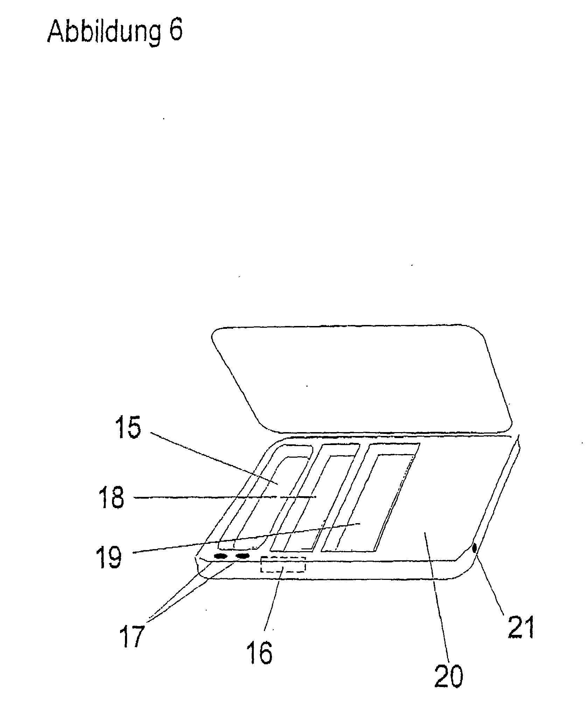

Nachfolgend wird ein bevorzugtes Ausführungsbeispiel der Erfindung anhand der Fig. 1 bis 6 beschrieben:
1. Die erfindungsgegenständliche rauchfreie Zigarette (nachstehend NICSTIC REFILL-SYSTEM) entspricht in der Größe exakt einer üblichen Zigarette.
2. NICSTIC REFILL-SYSTEM besteht aus 4 Teilen:
   2.1. aus einem austauschbarem, nicht wiederverwendbarem Teil, nämlich dem Filter (1), (optional) dem Nikometer - Verbrauchsanzeiger (2) und dem Nikotindepot incl. Aromastoffen (3) (Abbildung 1),
   2.2. aus einem wiederverwendbarem Teil (Abbildung 2), nämlich der aus Thermokunststoff beschichteten Ummantelung (4) mit einer Lufteinlassöffnung (5), Luftströmungskanälen (6), diese in Abbildung 2 im Längsschnitt und in Abbildung 3 im Querschnitt, einer Heizquelle (7) in Abbildung 3 sternförmig und in Abbildung 4 wellenförmig gestaltet und einem Auslöser/Zünder (8), wobei durch den kleineren Durchmesser (in Bezug auf den wiederverwendbaren Teil) und durch die Länge des Nikotindepots einerseits und einem entsprechend freigelassenen Raum im wiederverwendbaren Teil andererseits ein Aufstecken des austauschbaren Teils gemäß Abbildung 1 bis zum Filter bzw. dem Nikometer/ Verbrauchsanzeiger incl. Filter, dessen Durchmesser exakt dem wiederverwendbaren Teil entspricht, gewährleistet ist.
   2.3. alternativ aus einem wiederverwendbarem Teil (Abbildung 5), nämlich der aus Thermokunststoff bestehenden Hülse (9) mit einer Steckverbindung zur Aufladestation (10) in der Lufteinlassöffnung (11) zur Heizspirale (12), mit einem wärmespeichernden Medium (13) und Luftströmungskanälen (14), wobei durch den kleineren Durchmesser (in Bezug auf den wiederverwendbaren Teil) und durch die Länge des Nikotindepots einerseits und einem entsprechend freigelassenen Raum im wiederverwendbaren Teil andererseits ein Aufstecken des austauschbaren Teils gemäß Abbildung 1 bis zum Filter bzw. dem Nikometer/ Verbrauchsanzeiger incl. Filter, dessen Durchmesser exakt dem wiederverwendbaren Teil entspricht, gewährleistet ist
   2.4. und zusammen mit dem unter 2.3. abgehandelten Teil aus einer Vorrats- und Auflade-Station als Etui ausgestaltet (Abbildung 6), die die in dem wiederverwendbaren Teil (Abbildung 5) vorhandene Heizspirale (12) über die Steckverbindung (10) an das Aufladefach (15) anschließt und solange erwärmt, bis der Thermostat (16) den Aufheizungsvorgang beendet und gleichzeitig über die LED-Anzeige (17) - die zuvor eine rote Farbe anzeigte - durch das Erscheinen der grünen Farbe den Verwendungszeitpunkt signalisiert; des weiteren besteht das vierte Teil aus einem Vorratsfach (18) für zwei oder weitere Teile entsprechend der Abbildung 5, die nach dem Entnehmen des aufgeheizten wiederverwendbaren Teils in das Ladefach zum Aufheizen gelegt werden können und dann dem Raucher nach dem Verbrauch der ersten "Zigarette" sofort wieder zur Verfügung stehen und ein weiteres Vorratsfach (19) für die nicht wiederverwendbaren Teile (Abbildung 1); des weiteren enthält die Vorrats- und Auflade-Station einen Akku im Akkubereich (20), der mit einer Steckeröffnung zum Aufladen des Akkus (21) verbunden ist.
3. Statt das Nikotin beim Verbrennen des Tabaks freizusetzen, ist das Nikotin in einem Nikotindepot (3), das sich unmittelbar vor dem Filter befindet, gebunden und wird zusammen mit Tabakaromastoffen durch die erwärmte Luft freigesetzt und vom Konsumenten inhaliert.
4. Die Erwärmung der Luft erfolgt durch die in der Heizquelle gespeicherte Energie. Die Energie ist entweder in Form von Kristallisationswärme (7), die durch den Auslöser/Zünder (8) aktiviert wird, gespeichert oder wird von einem wärmespeichernden Medium (13), das durch die Heizspirale (12) ausreichend erwärmt wird, abgegeben. Die nach der Aktivierung entstehende Wärme ist ausreichend, um für ca. 1-3 Minuten erwärmte Luft zu inhalieren, die das einer (in einer vom Konsumenten individuell gewünschten/gewählten Menge) Zigarette entsprechende Nikotin freisetzen kann. Dabei ist gewährleistet, dass die Wärme über den Zeitraum von ca. 1-3 Minuten gleichmäßig abgegeben wird. Auch bei einer Unterbrechung der Inhalation entsteht durch die freigesetzte Kristallisationswärme/ Abgabewärme des wärmespeichernden Mediums keine Störung, schon gar keine Gefährdung, da die Wärme auch durch die Lufteinlassöffnung abgegeben werden kann.
5. Die Heizquelle (7) wird durch einen Stoff gespeist, der nach der Kristallisation und der Wärmeabgabe bis zu 3.000 Mal durch Wärmezuführung (durch ein Wasserbad in heißem/kochendem Wasser) wieder "aufgeladen" werden kann oder aus einem wärmespeichernden Medium (13), das durch Zuführung elektrischer Energie über Heizspiralen gem. Abbildung 6 kurz vor Rauchbeginn erwärmt wird, wobei durch einen Akku (wiederaufladbar an jeder Steckdose) eine Aufladung zur Durchführung jeweils von vielfachen Rauchvorgängen und insgesamt eine jahrelange Gebrauchsfähigkeit gewährleistet ist.

## Patentansprüche

1. Rauchfreie Zigarette bestehend aus zwei Teilen:
einem *wiederverwendbaren Teil* mit einer Kunststoff-Ummantelung, Lufteinlassöffnung (5) und einer Heizquelle (7), die so in die Ummantelung eingebracht ist, dass Luftströmungskanäle (6) entstehen, und
einem *austauschbaren*, *nicht wiederverwendbaren* Nikotin-Aroma-Depot (3), wobei die durch die Heizquelle freigesetzte Wärme, die vom Konsumenten durch die Luftströmungskanäle angesaugte Luft erwärmt und so das im Depot befindliche Nikotin und die Aromastoffe freisetzt, die vom Raucher aufgenommen werden, **dadurch gekennzeichnet, dass** die Kunststoff-Ummantelung eine Thermo-Kunststoff-Ummantelung ist mit aufgedampfter wärmerückstrahlender Beschichtung (4), und dass die Luftströmungskanäle (6) sternförmig oder wellenförmig sind.

2. Rauchfreie Zigarette nach Anspruch 1, **dadurch gekennzeichnet, dass** die Heizquelle (7) aus in Folie eingeschweißten Kristallen, die beim Kristallisierungsvorgang eine oder mehrere ca. 1- 3 Minuten dauernde Erwärmung(en) der Luft, die das Nikotin und die Aromastoffe im Nikotin-Aroma-Depot freisetzen kann, ausreichende Wärmemenge erzeugen kann, besteht und dass der Kristallisierungsvorgang durch ein Aktivierungsplättchen als Auslöser/Zünder (8) initiiert wird.

3. Rauchfreie Zigarette nach den Ansprüchen 1 + 2 **dadurch gekennzeichnet, dass** die Ladestation (Abbildung 6) mittels Thermostat (16) die zugeführte Energiemenge begrenzt.

4. Rauchfreie Zigarette nach den Ansprüchen 1+3 **dadurch gekennzeichnet, dass** gleichzeitig über eine LED-Anzeige (17) durch wechselnde Farben und/oder durch ein akustisches Signal dem Konsumenten signalisiert wird, ob er die rauchfreie Zigarette schon entnehmen kann.

5. Rauchfreie Zigarette nach den Ansprüchen 1, 3 und 4 **dadurch gekennzeichnet, dass** die Vorrats- und Auflade-Station als Etui (Abbildung 6) ausgestaltet ist, die zwei Vorratsfächer beinhaltet, das eine für zwei wiederverwendbare Teile (Abbildung 5) dergestalt, dass nach Entnahme des aufgeheizten wiederverwendbaren Teils sofort die nächsten wiederverwendbaren Teile der rauchfreien Zigarette aufgeheizt werden kann, das andere für nicht wiederverwendbare Teile (Abbildung 1).

## Claims

1. Smoke-free cigarette comprising two parts, namely a reusable part with a plastic envelope, air intake opening (5) and heating source (7) which is introduced into the envelope in such a way that air flow ducts (6) are formed and a replaceable, non-reusable nicotine aroma deposit (3), the heat released by the heating source heating the air sucked in by the consumer through the air flow ducts and in this way releasing the nicotine and aroma substances in the deposit and which are absorbed by the smoker, **characterized in that** the plastic envelope is a thermoplastic envelope with a vapour-deposited, heat-reflecting coating (4) and that the air flow ducts (6) are radial or wavy.

2. Smoke-free cigarette according to claim 1, **characterized in that** the heating source (7) comprises crystals welded into foil and which during the crystallization process can produce an adequate heat quantity in the form of one or more approximately 1 to 3 minute heatings of the air which can release the nicotine and the aroma substances in the nicotine aroma deposit and that the crystallization process is initiated by an activating platelet as an initiator/igniter (8).

3. Smoke-free cigarette according to claims 1 and 2, **characterized in that** the charging station (fig. 6) limits the energy quantity supplied by means of thermostat (16).

4. Smoke-free cigarette according to claims 1 and 3, **characterized in that** simultaneously by means of a LED display (17) and changing colours and/or by an acoustic signal the consumer is informed whether he can remove the smoke-free cigarette.

5. Smoke-free cigarette according to claims 1, 3 and 4, **characterized in that** the storage and charging station is constructed as a case (fig. 6), which has two storage compartments, one being for two reusable parts (fig. 5) so that following the removal of the heated, reusable part immediately the next reusable parts of the smoke-free cigarette can be heated and the other for non-reusable parts (fig. 1).

## Revendications

1. Cigarette sans fumée constituée de deux parties :
une partie réutilisable comportant une enveloppe en matière plastique, un orifice d'admission d'air (5) et une source de chauffage (7), qui est intégrée dans l'enveloppe de façon à engendrer des canaux de circulation d'air (6), et
un dépôt de nicotine et d'agents de saveur (3) interchangeable, non réutilisable, la chaleur dégagée par la source de chauffage chauffant l'air aspiré par le consommateur à travers les canaux de circulation d'air, et libérant ainsi la nicotine et les agents de saveur contenus dans le dépôt, qui sont absorbés par le fumeur,
**caractérisée en ce que** l'enveloppe en matière plastique est une enveloppe en matière thermoplastique avec un revêtement thermoréfléchissant (4) déposé en phase vapeur, et **en ce que** les canaux de circulation d'air (6) sont agencés en forme d'étoile ou en forme d'ondulations.

2. Cigarette sans fumée selon la revendication 1, **caractérisée en ce que** la source de chauffage (7) est constituée de cristaux soudés dans un film qui, lors du processus de cristallisation, peuvent engendrer une quantité de chaleur suffisante pour un ou plusieurs réchauffage(s) de l'air d'une durée de l'ordre de 1 à 3 minutes, qui peut (peuvent) libérer la nicotine et les agents de saveur contenus dans le dépôt de nicotine et d'agents de saveur, et **en ce que** le processus de cristallisation est initié par une pastille d'activation faisant office de déclencheur / allumeur (8).

3. Cigarette sans fumée selon les revendications 1 et 2, **caractérisée en ce que** la quantité d'énergie amenée au système de recharge (figure 6) est limitée au moyen d'un thermostat (16).

4. Cigarette sans fumée selon les revendications 1 et 3, **caractérisée en ce que**, par l'intermédiaire d'un affichage LED (17), il est simultanément signalé au consommateur par des couleurs changeantes et/ou un signal acoustique s'il peut déjà retirer la cigarette sans fumée.

5. Cigarette sans fumée selon les revendications 1, 3 et 4, **caractérisée en ce que** le système de réserve et de recharge est agencé sous la forme d'un étui (figure 6), comportant deux compartiments de réserve, l'un pour deux parties réutilisables (figure 5) de telle sorte que, après le retrait de la partie réutilisable chauffée, les parties réutilisables suivantes de la cigarette sans fumée puissent être immédiatement chauffées, et l'autre compartiment pour des parties non réutilisables (figure 1).
